(19) Europäisches Patentamt
European Patent Office
Office européen des brevets

(11) Publication number: **0 499 011 A1**

# (12) EUROPEAN PATENT APPLICATION

(21) Application number: **91810097.5**

(22) Date of filing: **12.02.91**

(51) Int. Cl.5: **C09K 11/06**, H05B 33/14, C07D 487/04, C09B 57/00, C08K 5/3415

(43) Date of publication of application: **19.08.92 Bulletin 92/34**

(84) Designated Contracting States: **GB**

(71) Applicant: **Japat Ltd**
**Klybeckstrasse 141**
**CH-4057 Basel(CH)**

(72) Inventor: **Matsumura, Michio, Dr.,**
**2-9-1-1073, Nishikoya,**
**Amagasaki-shi, Hyogo,(JP)**
Inventor: **Kudo, Tetsu, Dr.,**
**4-3-912, Mukogawa-cho,**
**Takarazuka-shi, Hyogo,(JP)**
Inventor: **Wooden, Gary, Dr.,**
**Gansmatt 351**
**CH-1716 Oberschrot(CH)**

(74) Representative: **Schluep, Hans-Peter et al**
**c/o CIBA GEIGY AG Patentabteilung**
**Postfach**
**CH-4002 Basel(CH)**

## (54) Organic electroluminescent element.

(57) Organic electroluminescent elements, comprising a compound of formula I

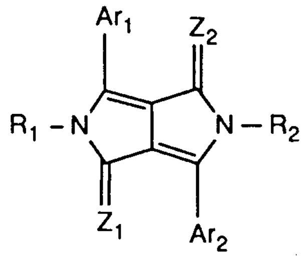

(I),

wherein $Z_1$ and $Z_2$ independently of each other stand for O or S, $R_1$ and $R_2$ independently of each other represent a hydrogen atom, an alkyl group having 1 to 18 carbon atoms, an alkenyl group having 3 to 18 carbon atoms and the double bond not being in the $C_1$-position or a phenylalkyl group having 1 to 5 carbon atoms in the alkyl, $Ar_1$ and $Ar_2$ independently of each other represent a 3-pyridyl or a 4-pyridyl residue or a group of the formula II

EP 0 499 011 A1

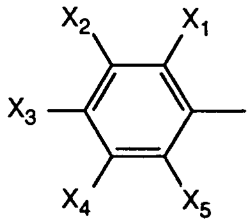

(II),

wherein $X_1$ and $X_5$ independently of each other represent a hydrogen atom, an alkyl group having 1 to 5 carbon atoms, an alkoxy group having 1 to 5 carbon atoms or halogen, and $X_2$, $X_3$ and $X_4$ independently of each other represent a hydrogen atom, an alkyl group having 1 to 5 carbon atoms, or an alkoxy group having 1 to 5 carbon atoms, or are dialkylamino having 1 to 5 carbon atoms per alkyl group, phenyl, cyano, trifluoromethyl or halogen, as a light emitting material can easily be produced and are distinguished by high stability, a long life time and a high brightness in light emission.

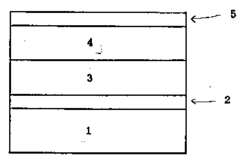


Fig.1

The present invention relates to an organic electroluminescent element to be used for various display devices.

Electroluminescent elements (hereinafter, referred to as EL elements) have been used for high quality word processors as displaying devices offering high quality pictures. Conventionally, EL elements are prepared by forming a light emitting layer containing inorganic materials, such as ZnS:Mn, on a glass substrate provided with a transparent electrode, such as ITO (Indium Tin Oxide), by means of the sputtering method or by coating a mixture prepared by dispersing the above light emitting material in a polymer matrix, followed by forming a counter electrode on said light emitting layer.

The inorganic EL elements are excellent in weather resistance, mechanical strength and the like, and because of this they can be used for a long time. However, since light emitting materials applicable to inorganic EL elements are limitted to the above mentioned materials, it is only possible to realize emission of monochromatic light, and as a result it is impossible to realize multi-colored or full-colored displaying. Moroever, inorganic EL elements have serious problems in that they cannot be driven unless an AC (alternating current) voltage of as high as 200 V is applied.

Accordingly, investigations have increasingly been carried out in order to solve the above described problems. In recent years, organic EL elements have been developed using organic light emitting materials. These organic EL elements now attract attention as an alternative to inorganic EL elements because they can be driven by applying a low DC (direct current) voltage, because there is practically an infinitely large possibility for selecting a light emitting material since it is an organic material, so that it is possible to emit light of any desired color, and furthermore, because it is very easy to produce such organic EL elements.

The principle of the above organic EL elements is based on the phenomena that electrons and positive holes, injected from a negative electrode and a positive electrode respectively, recombine in the above organic light emitting layer, thereby generating excitons, which are excited states thereof. The thus generated excitons diffuse in said light emitting layer, which then emits light when it returns to the ground state. Therefore, the wavelength of emitted light is only dependent on the difference in energy between the ground state and the excited states.

As is obvious from the above principle, it is not necessary to apply an AC voltage to organic EL elements. Moreover, it is possible to obtain light of any desired color in view of the sheer innumerable organic compounds, from which a suitable light emitting material can be selected.

In practice, organic EL elements are usually produced by forming a positive electrode, a positive hole transporting layer, an organic light emitting layer and a negative electrode in this order, thereby decreasing the threshold voltage for light emission. Organic EL elements comprising only an organic light emitting layer and two electrodes sandwiching it usually require application of a high voltage in order to emit light. The electrodes are usually formed by the vacuum deposition method or the sputtering method, and the positive hole transporting layer and the organic light emitting layer are formed by vacuum deposition. If necessary, an electron transporting layer is placed between the light emitting layer and the negative electrode. Whichever the case may be, light emission of about 1000 $cd/cm^2$ in intensity has been obtained upon application of a voltage of as low as 10 V or less.

In spite of the above described advantageous characteristics and possibilities, various restrictions are imposed on the choice of an organic light emitting material and as a result only a relatively small number of organic compounds are disclosed as potentially useful organic light emitting materials.

In general, it is believed that organic light emitting materials must satisfy the following conditions:

(1) They show strong fluorescence in the solid state.

(2) They form an amorphous layer (film) by vacuum deposition, which is a prerequisite for forming an even and pinholeless layer (film).

(3) When a hole transporting layer is formed, they do not form any exciplex therewith.

Condition (1) above can easily be understood when considering that electroluminescence ocurrs as a result of electrical excitation of the organic light emitting material, diffusion of generated excitons and thereafter deactivation to the ground state singlet. The only difference between fluorescence and electroluminescence is whether the material is excited through an electrical process or a photon process. The processes taking place thereafter are the same in both cases.

The above condition (2) is attributed to the fact that organic layers for an organic EL element, such as a positive hole transporting layer, a light emitting layer and an electron transporting layer are usually formed by vacuum deposition. The layers formed by means of vacuum deposition usually have a thickness of from 500 to 2000 Å, so that if pinholes are formed in said layers, disadvantageous effects occur, such as for example a short circuit and electrical breakdown around said pinholes. Crystalline organic light emitting materials usually yield an uneven rough film having a lot of pinholes when formed by means of vacuum deposition, so that they cannot be used in practice.

Condition (3) above is attributed to the fact that if an organic light emitting material forms an exciplex with a positive hole transporting material, the excited state once generated is quenched by interaction thereof and as a result, no EL emission is observed.

As organic light emitting materials satisfying the aforementioned conditions (1) to (3) and expected to be applicable to practical use, only the following compounds are known.

Japanese J. Appl. Phys. 27, L713 (1988):

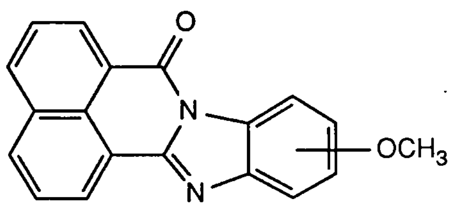

;

Proceedings for JAPAN DISPLAY 89, p. 708:

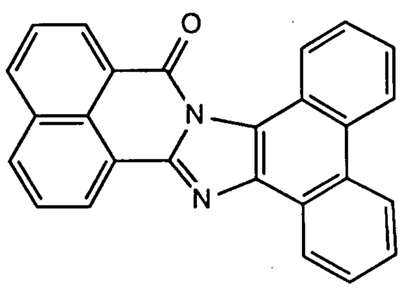

,

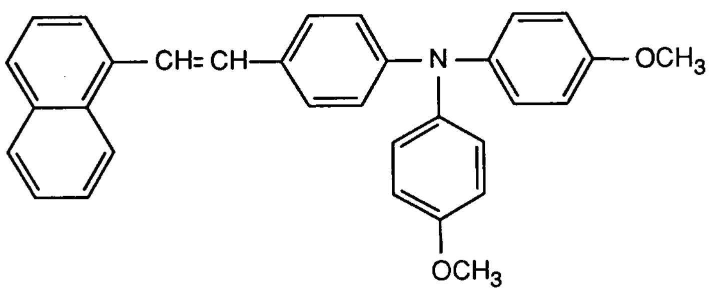

and

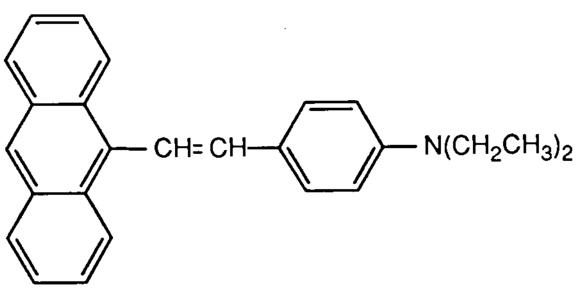

;

Abstract of the 36th Conference on Organic Materials for Electronics, Tokyo, p. 24 (1990):

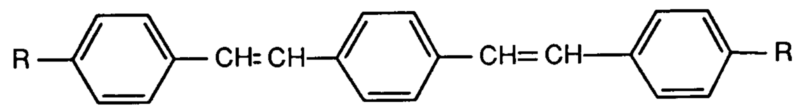

R = $CH_3$, $C_2H_5$

Chem. Lett. p. 189 (1990):

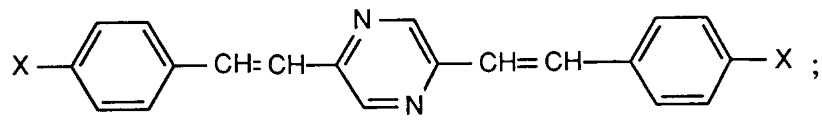

X = $C_2H_5$, $OCH_3$

J. Appl. Phys. 65, 3610 (1989):

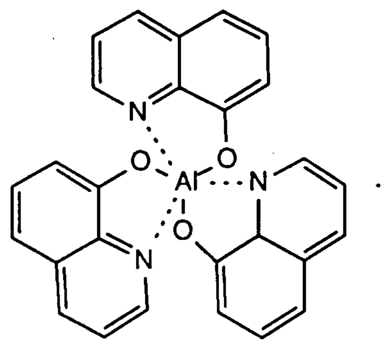

The above listed organic compounds each emit light at an intensity of about 1000 cd/cm$^2$ upon application of a DC voltage of as low as 10 V or less, when an adequate hole transporting layer or an electron transporting layer is employed.

However, the organic EL elements using any of the above compounds have a serious disadvantage in that their stability is not high enough for practical use. That is, since the light emitting layer is an amorphous layer, oxygen, moisture and the like can quite easily penetrate into said layer and as a result degrade the light emitting layer. This disadvantage is not restricted to the above compounds, but is an unavoidable problem occurring when using amorphous organic light emitting materials. Accordingly, a solution to the above serious problem is desired.

One object of the invention is to provide an organic electroluminescent (EL) element which is excellent in stability, life time and brightness in light emission, and which can be easily produced. This object is achieved by using a crystalline organic pigment as a light emitting material, the crystalline organic pigment being stable against oxygen and moisture, showing strong fluorescence in the solid state, forming a pinholeless smooth layer even in the case of vacuum deposition, and forming no exciplex with any positive hole transporting material.

The present invention relates to an organic EL element, comprising an organic pigment of formula I as a light emitting material

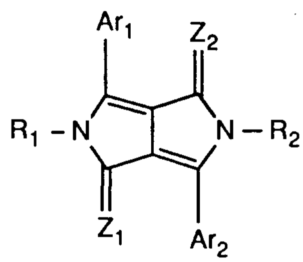

(I),

wherein $Z_1$ and $Z_2$ independently of each other stand for O or S, $R_1$ and $R_2$ independently of each other represent a hydrogen atom, an alkyl group having 1 to 18 carbon atoms, an alkenyl group having 3 to 18 carbon atoms and the double bond not being in the $C_1$-position or a phenylalkyl group having 1 to 5 carbon atoms in the alkyl, $Ar_1$ and $Ar_2$ independently of each other represent a 3-pyridyl or a 4-pyridyl residue or a group of the formula II

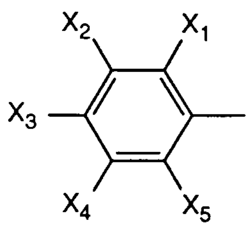

(II),

wherein $X_1$ and $X_5$ independently of each other represent a hydrogen atom, an alkyl group having 1 to 5 carbon atoms, an alkoxy group having 1 to 5 carbon atoms or halogen, and $X_2$, $X_3$ and $X_4$ independently of each other represent a hydrogen atom, an alkyl group having 1 to 5 carbon atoms, or an alkoxy group having 1 to 5 carbon atoms, or are dialkylamino having 1 to 5 carbon atoms per alkyl group, phenyl, cyano, trifluoromethyl or halogen.

The alkyl groups, the alkenyl groups and the alkyl residue of the alkoxy and dialkylamino groups can be straight chain or branched, straight chain groups being preferred. Examples of suitable alkyl groups are methyl, ethyl, propyl, isopropyl, n-, sec- and tert-butyl, pentyl, hexyl, octyl, decyl, dodecyl or octadecyl.

$Z_1$ and $Z_2$ are preferably the same and stand preferably for O.

$R_1$ and $R_2$ independently of each other are preferably hydrogen, $C_1$-$C_5$alkyl, $C_3$ to 5 alkenyl or benzyl and more preferably hydrogen, methyl, allyl or benzyl.

The substituents $X_2$, $X_3$ and $X_4$ independently of each other preferably represent a hydrogen atom, an alkyl group having 1 to 5 carbon atoms, methoxy, dimethylamino, phenyl, cyano, trifluoromethyl, chloro or bromo, and more preferably independently of each other are hydrogen, methyl, methoxy, phenyl, cyano or chloro.

The substituents $X_1$ and $X_5$ independently of each other preferably represent a hydrogen atom, an alkyl group having 1 to 5 carbon atoms, chloro, or methoxy and more preferably independently of each other are hydrogen, methyl or methoxy.

Preferred compounds of formula I are those, wherein $R_1$ and $R_2$ are the same and $Ar_1$ and $Ar_2$ are the same and are preferably a group of formula II.

The most preferred compounds of formula I are those, wherein $R_1$ and $R_2$ are each methyl, $X_1$ is hydrogen, methyl or methoxy and $X_2$, $X_3$, $X_4$ and $X_5$ are each hydrogen.

Fig. 1 is a cross sectional view of an organic EL element illustrating the fundamental structure of organic EL elements, wherein numeral 1 indicates a glass substrate, numeral 2 indicates an ITO electrode, numeral 3 indicates a positive hole transporting layer, numeral 4 indicates an organic light emitting layer and numeral 5 indicates a negative electrode.

Fig. 2(a) shows an ITO-provided glass substrate used for Examples 1, 2, 7 and 8. The shadowed portion was coated with a polycarbonate film.

Fig. 2(b) shows the process for preparing an EL element in Examples 1, 2, 7 and 8, wherein numeral 1 indicates a glass substrate, numeral 2 indicates an ITO electrode, numeral 3 indicates a polycarbonate insulating film, numeral 4 indicates a positive hole transporting layer, numeral 5 indicates an organic light emitting layer and numeral 6 indicates a negative electrode.

Fig. 3 shows an X-ray diffraction pattern obtained from the light emitting layer of Example 1.

Fig. 4 shows an X-ray diffraction pattern obtained from the light emitting layer of Example 7.

The present invention has been achieved based on the findings that a crystalline organic pigment of the above formula I shows strong fluorescence in the solid state, yields a smooth and pinholeless polycrystalline layer even by vacuum deposition and does not form an exciplex with any positive hole transporting material, and that an organic EL element produced by using such a pigment shows strong EL emission upon application of a low DC voltage, and the wavelength of said EL emission varies significantly depending on the substituents $R_1$, $R_2$, $X_1$ to $X_5$ and $Y_1$ to $Y_5$.

The EL element of the invention is excellent in stability against oxygen and moisture, and as a result, it has a long life time.

The compounds of formula I can be produced, for example, by the methods described in US 4,579,949 and US 4,585,878. Some compounds of formula I are also described in WO 90/1480.

The organic EL elements according to the invention can be produced as follows:

(1) Preparation of a substrate

The organic EL element of the invention employs an organic thin film as a light emitting layer, so that it is necessary to use a substrate of sufficient mechanical strength. The substrate may be either electroconducting or electrically insulating. In case of using an electroconducting substrate, a light emitting layer or a positive hole transporting layer is directly formed thereupon, while in case of using an electrically insulating substrate, an electrode is firstly formed thereupon and then a light emitting layer or a positive hole transporting layer is superposed.

The substrate may be either transparent, semi-transparent or opaque. However, in case of using a substrate as an indicating plane, the substrate must be transparent or semi-transparent.

Transparent electrically insulating substrates are, for example, inorganic compounds such as glass, quartz and the like, organic polymeric compounds such as polyethylene, polypropylene, polymethylmethacrylate, polyacrylonitrile, polyester, polycarbonate, polyvinylchloride, polyvinylalcohol, polyvinylacetate and the like. Each of these substrates can be transformed into a transparent electroconducting substrate by providing it with an electrode according to one of the methods described in the following section (2).

As examples of semi-transparent electrically insulating substrates, there are inorganic compounds such as alumina, YSZ (Yttrium Stabilized Zirconia) and the like, organic polymeric compounds such as polyethylene, polypropylene, polystyrene, epoxy resin and the like. Each of these substrates can be transformed into a semi-transparent electroconducting substrate by providing it with an electrode according to one of the methods described in the following section (2).

As examples of opaque electroconducting substrates, there are metals such as aluminum, indium, iron, nickel, zinc, tin, chromium, titanium, copper, silver, gold, platinum and the like, various electroplated metals, metallic alloys such as bronze, stainless steel and the like, semiconductors such as Si, Ge, GaAs and the like, electroconducting polymers such as polyaniline, polythiophene, polypyrrole, polyacetylene, polyparaphenylene and the like.

A substrate can be obtained by forming one of the above listed substrate materials to a desired dimension. It is preferred that the substrate has a smooth surface. Even if it has a rough surface, however, it will not cause any problem for practical use, provided that it has round unevenness having a curvature of not less than 20 $\mu$m. As for the thickness of the substrate, there is no restriction as far as it ensures sufficient mechanical strength.

(2) Formation of an electrode

In case of using an electrically insulating substrate, it is necessary to form an electrode thereupon. As examples of adequate materials for an electrode, there are metals such as gold, silver, copper, aluminum, indium, iron, zinc, tin, chromium, titanium, vanadium, cobalt, nickel, lead, manganese, tungsten and the like, metallic alloys such as magnesium/copper, magnesium/silver, magnesium/aluminum, aluminum/indium and the like, semiconductors such as Si, Ge, GaAs and the like, metallic oxides such as ITO, ZnO and the like, metallic compounds such as CuI and the like, and furthermore, electroconducting polymers such as polyacetylene, polyaniline, polythiophene, polypyrrole, polyparaphenylene and the like.

Of these electrode materials, metals, metallic alloys, metallic oxides and metallic compounds can be transformed into electrodes, for example, by means of the sputtering method. In the case of using a metal or a metallic alloy as a material for an electrode, the electrode can be formed also by the vacuum deposition method. In the case of using a metal or a metallic alloy as a material for forming an electrode, the electrode can be formed, furthermore, by the chemical plating method (see for example, Handbook of

Electrochemistry, pp 383-387, Maruzen, 1985).

In the case of using an electroconducting polymer, an electrode can be made by forming it into a film by means of anodic oxidation polymerization method onto a substrate which is previously provided with an electroconducting coating.

The thickness of an electrode to be formed on a substrate is not limited to a particular value, but, when the substrate is used as a light emitting plane, the thickness of the electrode is preferably within the range of from 10 to 100 Å, more preferably, within the range of 50 to 500 Å so as to ensure transparency.

(3) Formation of an organic light emitting layer

In the present invention, an organic light emitting layer is prepared by forming a thin film of a compound of formula I on an aforementioned electrode.

As methods for forming said thin film, there are, for example, the vacuum deposition method, the spin-coating method, the casting method, the LB (Langmuir-Blodgett) method and the like. Among these methods, the vacuum deposition method, the spin-coating method and the casting method are particularly preferred in view of ease in operation and cost.

In case of forming a thin film using a compound of formula I by means of the vacuum deposition method, the conditions under which the vacuum deposition is carried out are strongly dependent on the properties, shape and crystalline state of the compound. However, optimum conditions can be selected for example within the range of 100 to 400 ° C in temperature for the heating boat, -100 to 350 ° C in substrate temperature, $1x10^{-2}$ to $1x10^{-6}$ Torr in pressure and 0.01 to 60 Å/sec in deposition rate.

In an organic EL element, the thickness of the light emitting layer thereof is one of the factors determining its light emission properties. For example, if a light emitting layer is not sufficiently thick, a short circuit can ocurr quite easily between two electrodes sandwiching said light emitting layer, and therefore, no EL emission is obtained. On the other hand, if the light emitting layer is excessively thick, a large potential drop occurrs inside the light emitting layer because of its high electrical resistance, so that the threshold voltage for EL emission increases. Accordingly, it is necessary to limit the thickness of an organic light emitting layer within the range of from about 50 to 50000 Å. A preferable thickness is within the range of 100 to 5000 Å.

In the case of forming a light emitting layer by using the spin-coating method and the casting method, the coating can be carried out using a solution prepared by dissolving the compound of formula I in a concentration of 0.0001 to 90 % by weight in an appropriate organic solvent such as benzene, toluene, xylene, tetrahydrofuran, methyltetrahydrofuran, N,N-dimethylformamide, dichloromethane, dimethylsulfoxide and the like. Herein, the higher the concentration of the compound of formula I, the thicker the resulting film, while the lower the concentration, the the thinner the resulting film. However, if the concentration exceeds 90 % by weight, the solution is so viscous that it no longer permits forming a smooth and homogeneous film. On the other hand, if the concentration is less than 0.0001 % by weight, the efficiency of forming a film is too low to be economical. Accordingly, a preferred concentration of the compound represented by the general formula I is within the range of 0.01 to 80 % by weight.

In the case of using the above spin-coating or casting method, it is possible to further improve the homogeniety and mechanical strength of the resulting layer by adding a polymer binder in the solution for forming the light emitting layer. In principle, any polymer binder may be used, provided that it is soluble in a solvent in which the compound of formula I is dissolved. Examples of such polymer binders are polycarbonate, polyvinylalcohol, polymethylacrylate, polymethylmethacrylate, polyester, polyvinylacetate, epoxy resin and the like. A solution for forming a light emitting layer may have any concentrations of a compound of formula I, of a polymer binder and solvent. However, if the solid content composed of the polymer binder and the compound of formula I exceeds 99 % by weight, the fluidity of the solution is so low that is impossible to form a light emitting layer excellent in homogeniety. On the other hand, if the content of the compound of formula I is substantially smaller than that of the polymer binder, the electrical resistance of said layer is very large, so that it does not emit light unless a high voltage is applied thereto. Furthermore, since the concentration of the compound of formula I in the layer is small in this case, its light emission efficiency is relatively low. Therefore, the object of the invention cannot be achieved in this case, either. Accordingly, the preferred composition ratio of a polymer binder to a compound of formula I is within the range of about 10:1 to 1:50 by weight, and the solid content composed of both components in the solution is preferably within a range of about 0.01 to 80 % by weight, and more preferably, within the range of about 0.1 to 60 % by weight.

In the case of forming a light emitting layer by the spin-coating method or the casting method, the thickness of said layer may be selected in the same manner as in the cases of forming a light emitting layer

by the vacuum deposition method. That is, the thickness of the layer should be within the range of from 50 to 50000Å, and more preferably, within the range of 100 to 5000 Å for the same reason as in the case of vacuum deposition.

(4) Formation of a positive hole transporting layer

Although the organic EL element of the invention can emit light upon application of a voltage of as low as 20 V even in cases that it has such a simple structure as a substrate/ an electrode/ a light emitting layer/ an electrode, the threshold voltage for EL emission can be decreased by adding an organic positive hole transporting layer.

Examples of substances capable of being used as a positive hole transporting material include the following organic compounds:

- a TPD compound disclosed in J. Amer. Chem. Soc. 90, 3925 (1968)

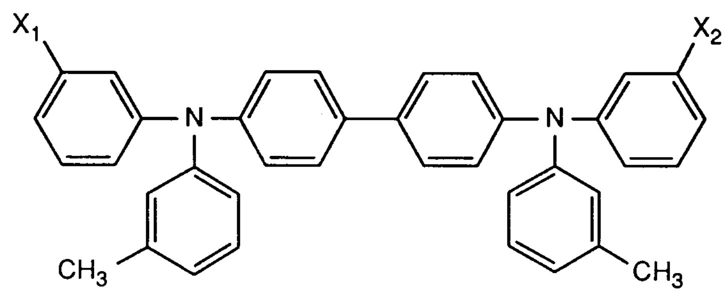

wherein $X_1$ and $X_2$ each represent a hydrogen atom or a methyl group;

- commercially available polyvinylcarbazole (PVK)

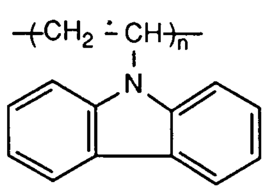

;

- a compound disclosed in J. Appl. Phys. 65(9), 3610 (1989)

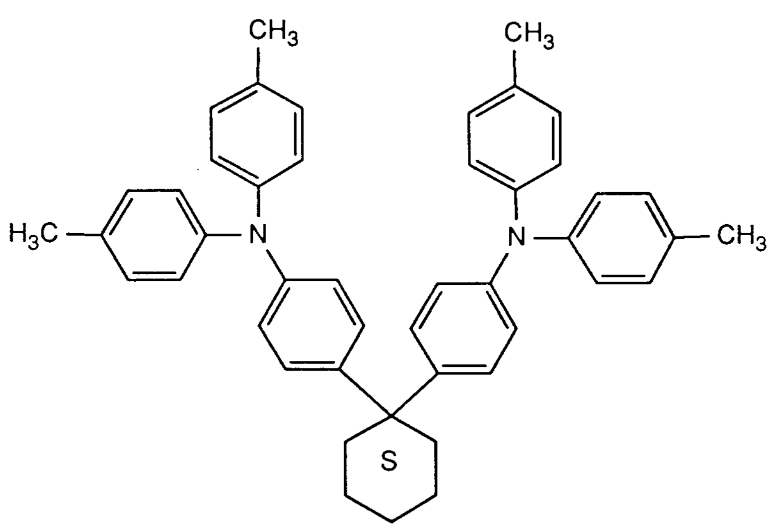

;

- a stilbene based compound

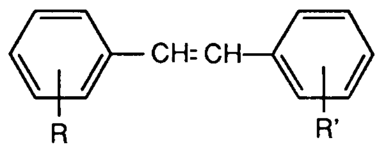 ;

- a hydrazone based compound

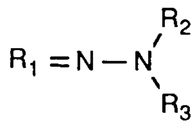

and the like.

Compounds to be used as a positive hole transporting material are not restricted to the above listed compounds. Any compound having a property of transporting positive holes can be used as a positive hole transporting material.

A positive hole transporting layer can be formed by preparing an organic film containing at least one positive hole transporting material on the aforementioned light emitting layer. The positive hole transporting layer can be formed by the vacuum deposition method, the spin-coating method, the casting method, the LB method and the like. Of these methods, the vacuum deposition method, the spin-coating method and the casting method are particularly preferred in view of ease and cost.

In the case of using the vacuum deposition method, the conditions for deposition may be chosen in the same manner as described above for the formation of a light emitting layer. If it is desired to form a positive hole transporting layer comprising more than one positive hole transporting material, the coevaproation method can be employed using the desired compounds.

In the case of forming a positive hole transporting layer by the spin-coating method or the casting method, the layer can be formed under the conditions described above for the formation of the light emitting layer. The same pertains for the preferred conditions.

As in the case of forming a light emitting layer using a solution containing a polymer binder, a smoother and more homogeneous positive hole transporting layer can be formed by using a solution containing a binder and at least one positive hole transporting material. The coating using such a solution can be performed in the same manner as in cases of forming a light emitting layer using a polymer binder. Again, any polymer binder may be used, provided that it is soluble in a solvent in which at least one positive hole transporting material is dissolved. Examples of appropriate polymer binders and of appropriate and preferred concentrations are given above when describing the formation of the light emitting layer.

The thickness of a positive hole transporting layer should be up to 50000 Å. The upper limit of the thickness is set to 50000 Å, because if the layer has a thickness of more than 50000 Å, a serious potential drop ocurrs in the layer. A preferable thickness is up to 5000 Å.

(5) Formation of the counter electrode

It is necessary to form a counter electrode on the organic light emitting layer described in the above section (3), or on the positive hole transporting layer described in section (4). Materials for forming an electrode and methods for forming it are described in section (2) above. However, in order not to spoil the above layers, it is preferred to form a counter electrode by a dry process. That is, methods for forming an electrode should be limited to the vacuum deposition method and the sputtering method. It is preferred to form an electrode by depositing a metal, a metallic alloy, a metallic oxide, a metallic compound and the like by using the vacuum deposition method or the sputtering method.

In the above example of embodiments of the present invention, an organic EL element is produced by a process comprising the steps of (1), (2), (3), (4) and (5) in this order, wherein the step (2) is not necessary if the substrate is electroconducting, and the step (4) is not necessary if no positive hole transporting layer is formed. However, the structure of the organic EL element and the method for producing the same according to the invention are not limited to the above described ones. For example, the steps of (3) and (4) may be interchanged; that is, another organic EL element can be produced by the steps of forming a

positive hole transporting layer on an electroconducting substrate or a substrate provided with an electrode, then forming an organic light emitting layer on said positive hole transporting layer, and thereafter forming a counter electrode on said organic light emitting layer. In this case, the positive hole transporting layer and the organic light emitting layer are formed by using the methods and materials as described in (4) and (3), respectively. That is, the organic EL element of the invention may have any of the following structures: a substrate/an electrode/an organic light emitting layer/an electrode; a substrate/an electrode/an organic light emitting layer/a positive hole transporting layer/an electrode and a substrate/an electrode/a positive hole transporting layer/an organic light emitting layer/an electrode. Moreover, an electron transporting layer having a property of transporting electrons may be inserted between a pair of adjacent electrode and light emitting layer. That is, the organic EL element of the invention may further have any of the following structures: a substrate/an electrode/an electron transporting layer/an organic light emitting layer/an electrode; a substrate/an electrode/an organic light emitting layer/an electron transporting layer/an electrode; a substrate/an electrode/an electron transporting layer/an organic light emitting layer/a positive hole transporting layer/an electrode; and a substrate/an electrode/a positive hole transporting layer/an organic light emitting layer/an electron transporting layer/an electrode.

Examples of suitable electron transporting materials for the formation of an electron transporting layer are:

- an oxydiazole derivative disclosed in JAPAN DISPLAY 89, p. 708

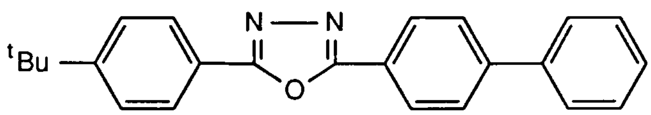
,

- a perylene tetracarboxylic acid derivative disclosed in Appl. Phys. Lett. 48, (2) 183 (1986)

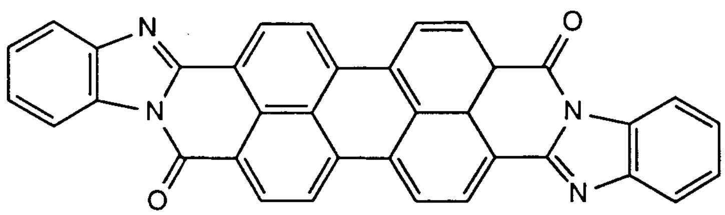

and the like.

An electron transporting layer can be formed according to the same process as described in section (3) above. That is, the electron transporting layer can be formed by such methods as the vacuum deposition method, the spin-coating method, the casting method and the like as in the cases of forming a light emitting layer or a positive hole transporting layer.

As in the case of forming a light emitting layer or a positive hole transporting layer by using a solution containing a polymer binder, a smoother and a more homogenious electron transporting layer can be formed by using a solution containing a binder and at least one electron transporting material.

The thickness of an electron transporting layer should be up to 50000 Å for the same reasons as outlined above for the positive hole transporting layer. The preferable thickness of the electron transporting layer is up to 5000 Å, more preferably, 50 to 3000 Å.

As explained above, the organic EL element according to the present invention can be produced at various embodiments. Because of the flexibility in selecting a structure of an EL element, and because of many ways in selecting materials and preparation methods for forming electrodes, an organic light emitting layer, a positive hole transporting layer and/or an electron transporting layer, the organic EL element can be produced at any desired embodiment according to the invention. Furthermore, it is possible to dope an organic light emitting layer with another fluorescent compound. In such a case, excited states of the light emitting layer (excitone) are transferred to the doping molecules and as a result, the doping molecules emit light. In order to achieve this, it is necessary that the excitation energy of the doped molecules is lower than that of the light emitting material, otherwise, the excitation energy of the light emitting layer cannot be transferred. Therefore, it is possible to shift the wavelength of emitted light to a longer wavelength by doping. In such a case, the organic light emitting layer does not act as a light emitting layer, but as a

medium transferring its excitation energy to doped molecules.

In the present invention, when a positive hole transporting layer and/or electron transporting layer is formed, the electrode adjacent to the positive hole transporting layer is used as a positive electrode, and the counter electrode is a negative electrode; likewise the electrode adjacent to the electron transporting layer is used as a negative electrode, and the counter electrode is a positive electrode. Light emission is obtained by applying a DC voltage between positive and negative electrodes in the forward direction. Not only a DC voltage but also an AC voltage can be applied. However, in such a case, the EL element emits light only during half the period in which voltage is applied in the forward direction. As described in section (2) above, no limitation is imposed on the materials to be used as an electrode, as far as they provide electroconductivity. However, depending on the combination of the organic layer and the adjacent electrode, there often occurs the formation of "Schottoky barriers" and the like, resulting in an increased threshold voltage for light emission. In order to avoid such undesirable effects, it is preferred to use a metal having a high work function, such as gold, silver, copper and the like, a metallic oxide having a high work function, such as ITO, ZnO and the like, a metallic compound having a high work function, such as CuI and the like, or an electroconducting polymer, such as polypyrrole, polythiophene, polyaniline, polyacetylene and the like as a material for forming the positive electrode. Preferred materials for the formation of a negative electrode are Al, In, Mg/Cu, Mg/Ag and the like.

The EL element of the present invention will be described in more detail with reference to the following examples.

Example 1: A sheet of commercially available glass plate provided with ITO (Indium Tin Oxide) is cut into pieces having a dimension of 75 mm x 15 mm, each of which is washed in an ultrasonic bath using an aqueous solution containing a detergent. They are subsequently rinsed with flowing distilled water for 60 minutes, dried in an oven and used as substrates. One of the thus obtained substrates is coated with a dichloromethane solution containing polycarbonate at a concentration of 6 % by weight by using the dip-coating method except the part on which a light emitting layer was formed and the part on which a lead was attached, as shown in Fig. 2(a), to form an insulating layer for preventing short circuit or electrical breakdown, which are liable to ocurr when a lead is attached to a negative electrode.

Next, a mask made of aluminum having an aperture larger in size by about 1 mm at each peripheral than the part on which the light emitting layer is formed is attached to the above substrate, which is then attached to a substrate holder of a commercially available vacuum deposition apparatus (made by ULVAC, Type VPC-260) to form a positive hole transporting layer. The positive hole transporting layer is formed using a TPD compound represented by the following formula

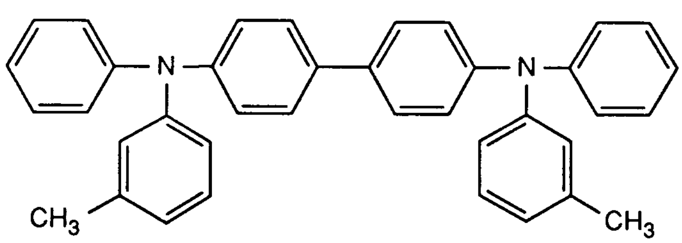

.

Said compound is vacuum deposited by means of resistance heating under the conditions that the substrate is kept at room temperature, the pressure inside the bell jar is $5 \times 10^{-5}$ Torr and the deposition rate is 11 Å/sec, to thereby form a positive hole transporting layer of 2000 Å in thickness.

Then, the bell jar is opened to atmosphere, and the boat containing TPD is replaced with another boat containing a light emitting compound represented by the formula

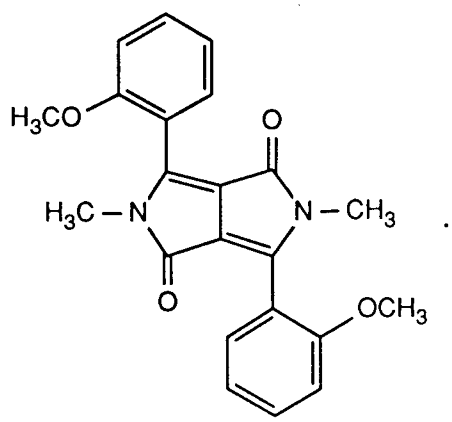

A glass slide having a dimension of 75 mm x 25 mm is also attached to the above mentioned substrate holder in order to form a vacuum deposited layer for examining the crystallinity of the thus formed layer. The above compound is vacuum deposited similarly by the resistance heating method under the conditions that the substrate is kept at room temperature, the pressure inside the bell jar 5 x $10^{-5}$ Torr and the desposition rate is 8 Å/sec to a thickness of 1500 Å, to thereby form a light emitting layer on the aforementioned positive hole transporting layer formed on the ITO substrate, and on the above glass slide.

After opening the bell jar to atmosphere, the ITO substrate and the glass slide are taken out of the vacuum deposition equipment. The vacuum deposited layer formed on the glass slide is subjected to X-ray diffraction analysis using an X-ray diffractometer, Type Rad IIC, manufactured by Rigaku Denki Co. After removing the aforementioned mask made of aluminium, an aluminium plate slightly larger in size than the part for attaching lead was attached to said part of the ITO substrate so that said part is covered with the plate. The ITO substrate is then attached to a substrate holder of another vacuum deposition equipment Type EBH-6 (ULVAC), whereafter an aluminium layer having a thickness of 250 Å is formed thereupon by means of resistance heating under the conditions that the substrate is kept at room temperature, the pressure inside the bell jar is 2 x $10^{-6}$ Torr and the deposition rate is 5 Å/sec.

Next, the bell jar is opened to atmosphere, and the sample is taken out of the vacuum deposition equipment. A lead is attached to each electrode using silver paste, to thereby complete an example of the organic EL element of the invention.

The above described process is shown in Fig. 2(b).

A result obtained from the aforementioned X-ray diffraction analysis is shown in Fig. 3. This figure reveals that the organic EL element of the invention has a crystalline organic light emitting layer, and not an amorphous one. Furthermore, the same sample as that subjected to X-ray diffraction analysis is examined by SEM (Scanning Electron Microscope) observation. It is found that no pinholes are formed, and that the above organic light emitting layer has a structure which comprises densely packed micro crystals free from pinholes.

Upon application of a voltage of 10 V in the forward direction, the EL element of this example emits light of a yellowish green color. When the luminance is measured using a commercially available light meter (produced by Minolta Co.), the EL element shows luminance of 403 cd/m$^2$ and current density of 71 mA/cm$^2$ at an applied voltage of 12 V, which are enhanced to 962 cd/m$^2$ and 221 mA/cm$^2$, respectively, when the applied voltage is increased to 14 V.

The above organic EL element is kept at room temperature under normal humidity for 60 days in order to examine its stability. After this examination, no deterioration in light emitting characteristics is observed.

Example 2: Example 1 is repeated by using stilbene as positive hole transporting material.

Upon application of a voltage of 11 V in the forward direction, the EL element of this example emits light of a yellowish green color. The EL element shows luminance of 382 cd/m$^2$ and current density of 67 mA/cm$^2$ at an applied voltage of 13 V, which are enhanced to 851 cd/m$^2$ and 193 mA/cm$^2$, respectively, when the applied voltage is increased to 15 V.

The above organic EL element is kept at room temperature under normal humidity for 60 days in order to examine its stability. After this examination, no deterioration in light emitting characteristics is observed.

Example 3: An ITO substrate prepared in Example 1 is used. Using a dichloromethane solution containing 1 % by weight of polycarbonate and 1 % by weight of TPD, a positive hole transporting layer having a thickness of 2000 Å is formed on the ITO substrate by means of the spin coating method.

Next, using a dichloromethane solution containing 1 % by weight of polycarbonate and 1 % by weight

of the light emitting compound of example 1, a light emitting layer of 3000 Å in thickness is formed on the above position hole transporting layer by means of the spin coating method. Subsequently, peripheral parts of the ITO substrate, where no film is formed during the above two spin coating steps, are covered with aluminum sheets. The substrate is attached to a substrate holder of a commercially available vacuum deposition equipment Type EBH-6 (ULVAC), and a negative electrode is formed on the aforementioned light emitting layer by vacuum depositing aluminum to a thickness of 300 Å under the conditions that the substrate is kept at room temperature, the pressure inside the bell jar is 2 x $10^{-6}$ Torr and the deposition rate is 5 Å/sec.

After opening the bell jar to atmosphere, the sample is taken out of the vacuum deposition equipment. The aforementioned aluminum sheets are removed, and a lead was attached to each of the electrodes to thereby complete an example of the EL element of the invention.

Upon application of a voltage of 23 V in the forward direction, the EL element emits light of a yellowish green color. Upon application of a voltage of 30 V, the luminance of light emission is 206 cd/$m^2$ and the current density is 59 mA/$m^2$.

Example 4: An ITO substrate prepared in Example 1 is used. Using a tetrahydrofuran solution containing 1 % by weight of polycarbonate and 1 % by weight of stilbene, a positive hole transporting layer of 2200 Å in thickness is formed on the ITO substrate by means of the spin coating method. Next, using a tetrahydrofuran solution containing 1 % by weight of polycarbonate and 1 % by weight of a light emitting compound of example 1, a light emitting layer having a thickness of 2500 Å is formed on the above positive hole transporting layer by means of the spin coating method. Subsequently, peripheral parts of the ITO substrate, on which no film is formed during the above two spin coating stepts, are covered with aluminum sheets. The sample is attached to a substrate holder of a commercially available vacuum deposition equipment Type EBH-6 (ULVAC), and then a negative electrode having a thickness of 300 Å is formed on the aforementioned light emitting layer by vacuum depositing aluminum thereto under the conditions that the substrate is kept at room temperature, the pressure inside the bell jar is 2 x $10^{-6}$ Torr and the deposition rate 5 Å/sec.

After opening the bell jar to atmosphere, the sample is taken out of the vacuum deposition equipment. The aforementioned aluminum sheets are removed from the sample, and then a lead is attached to each of the electrodes to thereby complete an example of the EL element of the invention.

When a voltage of 25 V is applied to the EL element in the forward direction, it emits light of a yellowish green color. Upon application of a voltage of 33 V, the EL element shows luminance of 172 cd/$m^2$ and current density of 48 mA/$cm^2$.

Example 5: A commercially available glass slide having a dimension of 75 mm x 25 mm is washed in an ultrasonic bath using an aqueous solution containing a detergent. It is subsequently rinsed with flowing distilled water for 60 minutes, dried in an oven and used as a substrate. In order to provide the glass slide with electroconductivity, an aluminum film of 400 Å in thickness is formed on one side of said substrate by means of vacuum deposition using a commercially available vacuum deposition equipment Type EBH-6 (ULVAC) under the conditions that the substrate is kept at room temperature, the pressure inside the bell jar was 3 x $10^{-6}$ Torr and the deposition rate was 10 Å/sec.

Next using an acetone solution containing 2 % by weight of polymethylmethacrylate and 2% by weight of the light emitting compound of example 1, a light emitting layer having a thickness of 3000 Å is formed on the above aluminum film by means of the spin coating method. Subsequently, using an acetone solution containing 2 % by weight of polymethylmethacrylate and 2 % by weight of a positive hole transporting compound

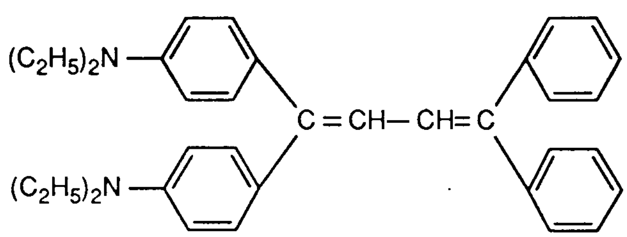

a positive hole transporting layer of 2000 Å in thickness is formed on the above light emitting layer by means of the spin coating method.

Then, peripheral parts of the ITO substrate, on which no film is formed during the above mentioned two

spin coating steps, are covered with aluminum sheets. The substrate is attached to a substrate holder of the vacuum deposition equipment which is used for forming the aluminum film, and a gold electrode having a thickness of 250 Å is formed on the above positive hole transporting layer by means of vacuum deposition under the conditions that the substrate is kept at room temperature, the pressure inside the bell jar is 2 x $10^{-6}$ Torr and the deposition rate is 1 Å/sec.

After opening the bell jar to atmosphere, the sample is taken out of the vacuum deposition equipment. The aforementioned aluminum sheets are removed from the substrate, and a lead is attached to each of the electrodes to thereby complete the EL element of the invention. When a voltage of 35 V is applied to the EL element in the forward direction, it emits light of a yellowish green color. The EL element shows luminance of 120 cd/$m^2$ and current density of 48 mA/$cm^2$ upon application of 41 V.

Example 6: A glass slide is coated with aluminum and with the light emitting layer as described in example 5 above. Subsequently, using an acetone solution containing 2 % by weight of polymethylmethacrylate and 2 % by weight of TPD, a positive hole transporting layer having a thickness of 2400 Å is formed on the light emitting layer by means of the spin coating method. The EL element is further prepared as described in example 5. When a voltage of 28 V is applied to the EL element in the forward direction, it emits light of a yellowish green color. The EL element shows luminance of 146 cd/$m^2$ and current density of 52 mA/$cm^2$ upon application of a voltage of 43 V.

Example 7: Example 1 is repeated by using a light emitting compound of the formula

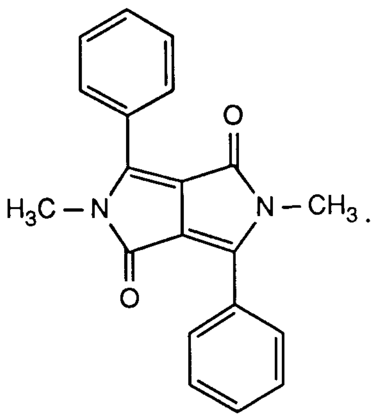

A result obtained from the X-ray diffraction analysis of the light emitting layer vacuum deposited on a glass slide as in example 1 is shown in Fig. 4. This figure shows that the organic EL element of the invention has a crystalline organic light emitting layer, and not an amorphous one. Furthermore, the same sample as that is subjected to X-ray diffraction analysis is examined by SEM (Scanning Electron Microscope type JSM-5200 manufactured by Nippon Denshi K.K.) observation. It is found that no pinholes are formed, and that the above organic light emitting layer has a structure comprising densely packed micro crystals free from pinholes.

Upon application of a voltage of 9 V in the forward direction, the EL element emits light of a yellowish red color. The EL element shows luminance of 392 cd/$m^2$ and current density of 82 mA/$cm^2$ at an applied voltage of 12 V, which are enhanced to 883 cd/$m^2$ and 203 mA/$cm^2$, respectively, when the applied voltage is increased to 13 V.

The above organic EL element is kept at room temperature under normal humidity for 60 days in order to examine its stability. After this examination, no deterioration in light emitting characteristics is observed.

Example 8: Example 7 is repeated using stilbene as a positive hole transporting material.

Upon application of a voltage of 11 V in the forward direction, the EL element of this example emits light of a yellowish red color. The EL element shows luminance of 371 cd/$m^2$ and current density of 73 mA/$cm^2$ at an applied voltage of 14 V, which is enhanced to 691 cd/$m^2$ and 198 mA/$cm^2$, respectively, when the applied voltage is increased to 16 V.

The above organic EL element is kept at room temperature under normal humidity for 60 days in order to examine its stability. After this examination, no deterioration in light emitting characteristics is observed.

Example 9: An ITO substrate prepared in Example 7 is used as a substrate. Using a dichloromethane solution containing 1 % by weight of polycarbonate and 1 % by weight of TPD, a positive hole transporting layer having a thickness of 1900 Å is formed on the above ITO substrate by means of the spin-coating

method.

Next using a dichloromethane solution containing 1 % by weight of polycarbonate and 1 % by weight of the light emitting compound of example 7 a light emitting layer of 2800 Å in thickness is formed by means of the spin coating method. Subsequently, an aluminum layer having a thickness of 300 Å is applied as described in example 1 and a lead is attached to each of the electrodes to thereby complete the EL element of the invention.

Upon application of a voltage of 25 V in the forward direction, the EL element emits light of a yellowish red color. When a voltage of 31 V is applied to the EL element, it shows luminance of 188 cd/$m^2$ and current density of 60 mA/$cm^2$.

Example 10: An ITO substrate prepared in Example 7 is used as a substrate. Using a tetrahydrofuran solution containing 1 % by weight of polycarbonate and 1 % by weight of stilbene, a positive hole transporting layer of 2100 Å in thickness is formed on the ITO substrate by means of the spin coating method. Next, using a tetrahydrofuran solution containing 1 % by weight of polycarbonate and 1 % by weight of the light emitting compound of example 7 a light emitting layer having a thickness of 3200 Å is formed by means of the spin coating method. Subsequently an aluminum layer is applied and a lead was attached to the electrodes as described in example 1.

When a voltage of 27 V is applied to the EL element in the forward direction, it emits light of a yellowish red color. Upon application of a voltage of 33 V, the EL element shows luminance of 190 cd/$m^2$ and current density of 56 mA/$cm^2$.

Example 11: Example 5 is repeated using the light emitting compound of example 7.

When a voltage of 37 V is applied to the EL element in the forward direction, it emits light of a yellowish red color. The EL element shows luminance of 108 cd/$m^2$ and current density of 45 mA/$cm^2$ upon application of 43 V.

Example 12: Example 11 is repeated except that by using an acetone solution containing 2 % by weight of polymethylmethacrylate and 2 % by weight of the aforementioned TPD, a positive hole transporting layer having a thickness of 2400 Å is formed on the above light emitting layer by means of the spin coating method. When a voltage of 31 V is applied to the EL element in the forward direction, it emits light of a yellowish red color. The EL element shows luminance of 125 cd/$m^2$ and current density of 51 mA/$cm^2$ at an applied voltage of 36 V.

## Claims

**1.** An organic electroluminescent element, comprising a compound of formula I

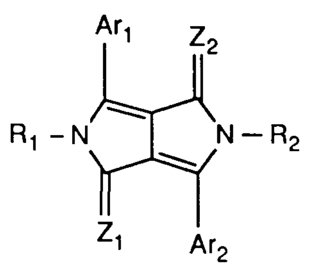

(I),

wherein $Z_1$ and $Z_2$ independently of each other stand for O or S, $R_1$ and $R_2$ independently of each other represent a hydrogen atom, an alkyl group having 1 to 18 carbon atoms, an alkenyl group having 3 to 18 carbon atoms and the double bond not being in the $C_1$-position or a phenylalkyl group having 1 to 5 carbon atoms in the alkyl, $Ar_1$ and $Ar_2$ independently of each other represent a 3-pyridyl or a 4-pyridyl residue or a group of the formula II

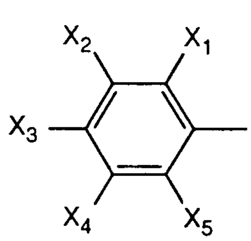

(II),

wherein $X_1$ and $X_5$ independently of each other represent a hydrogen atom, an alkyl group having 1 to 5 carbon atoms, an alkoxy group having 1 to 5 carbon atoms or halogen, and $X_2$, $X_3$ and $X_4$ independently of each other represent a hydrogen atom, an alkyl group having 1 to 5 carbon atoms, or an alkoxy group having 1 to 5 carbon atoms, or are dialkylamino having 1 to 5 carbon atoms per alkyl group, phenyl, cyano, trifluoromethyl or halogen, as a light emitting material.

**2.** An organic electroluminescent element according to claim 1, wherein $R_1$ and $R_2$ independently of each other are hydrogen, an alkyl group having 1 to 5 carbon atoms, an alkenyl group having 3 to 5 carbon atoms or benzyl.

**3.** An organic electroluminescent element according to claim 1 or 2, wherein $X_2$, $X_3$ and $X_4$ independently of each other represent a hydrogen atom, an alkyl group having 1 to 5 carbon atoms, methoxy, dimethylamino, phenyl, cyano, trifluoromethyl, chloro or bromo.

**4.** An organic electroluminescent element according according to any of claims 1-3, wherein $X_1$ and $X_5$ independently of each other represent a hydrogen atom, an alkyl group having 1 to 5 carbon atoms, chloro or methoxy.

**5.** An organic electroluminescent element according to any of claims 1-4, wherein $R_1$ and $R_2$ independently of each other are hydrogen, methyl, allyl or benzyl.

**6.** An organic electroluminescent element according to any of claims 1-5, wherein $X_2$, $X_3$ and $X_4$ independently of each other are hydrogen, methyl, methoxy, phenyl, cyano or chloro.

**7.** An organic electroluminescent element according to any of claims 1-6, wherein $X_1$ and $X_5$ independently of each other are hydrogen, methyl or methoxy.

**8.** An organic electroluminescent element according to any of claims 1-7, wherein $R_1$ and $R_2$ are the same, $Z_1$ and $Z_2$ are the same, $Ar_1$ and $Ar_2$ are the same and are preferably a group of the formula II.

**9.** An organic electroluminescent element according to any of claims 1-8, wherein $R_1$ and $R_2$ are each methyl, $X_1$ is hydrogen, methyl or methoxy and $X_2$, $X_3$, $X_4$ and $X_5$ are each hydrogen.

**10.** An organic electroluminescent element according to any of claims 1-9, wherein $Z_1$ and $Z_2$ are each oxygen.

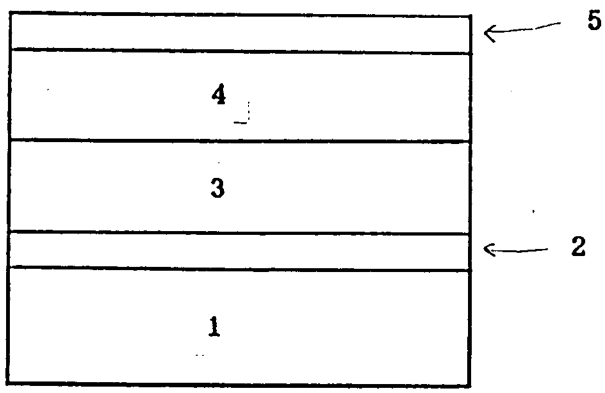


Fig.1

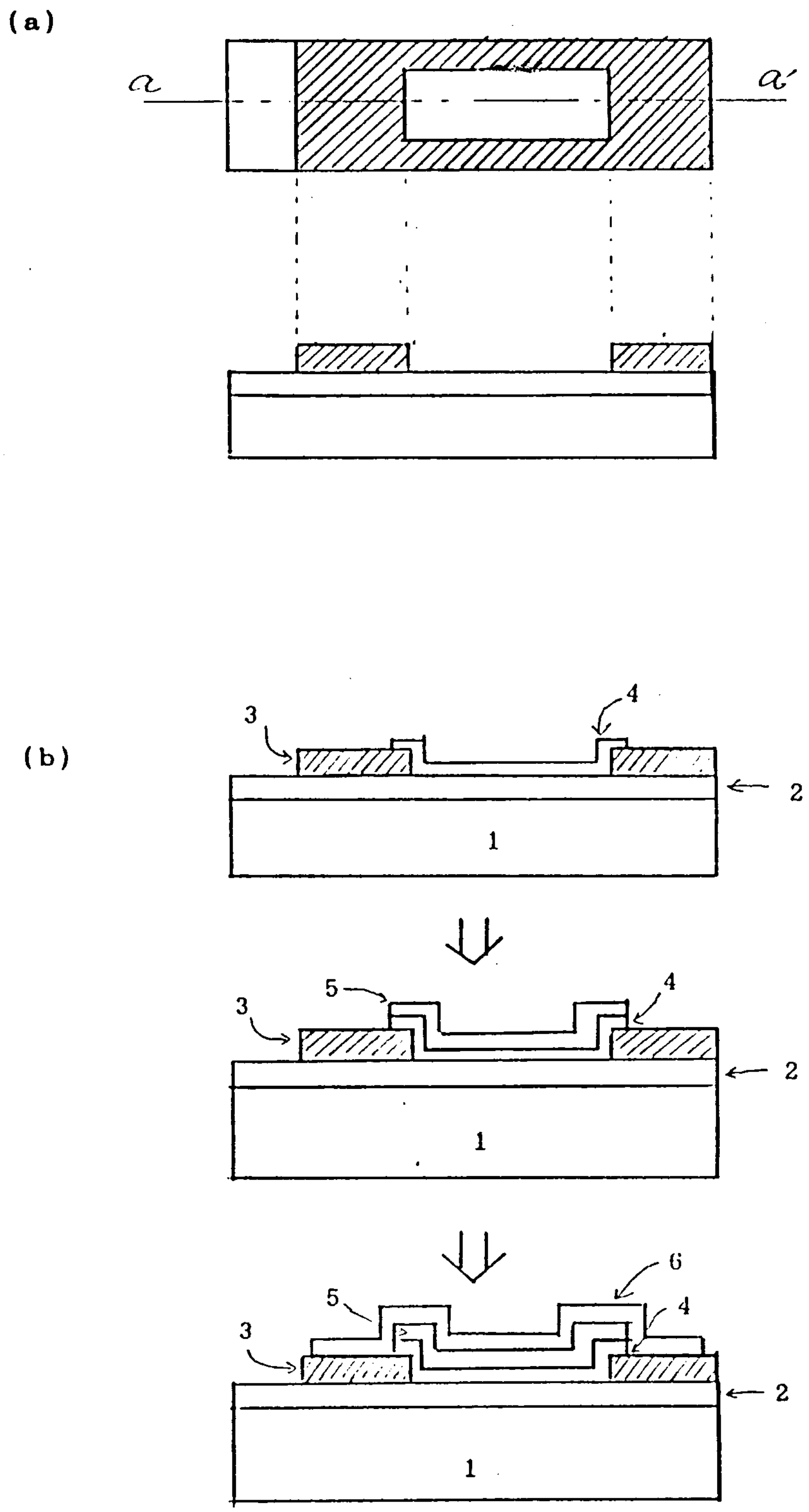


Fig.2

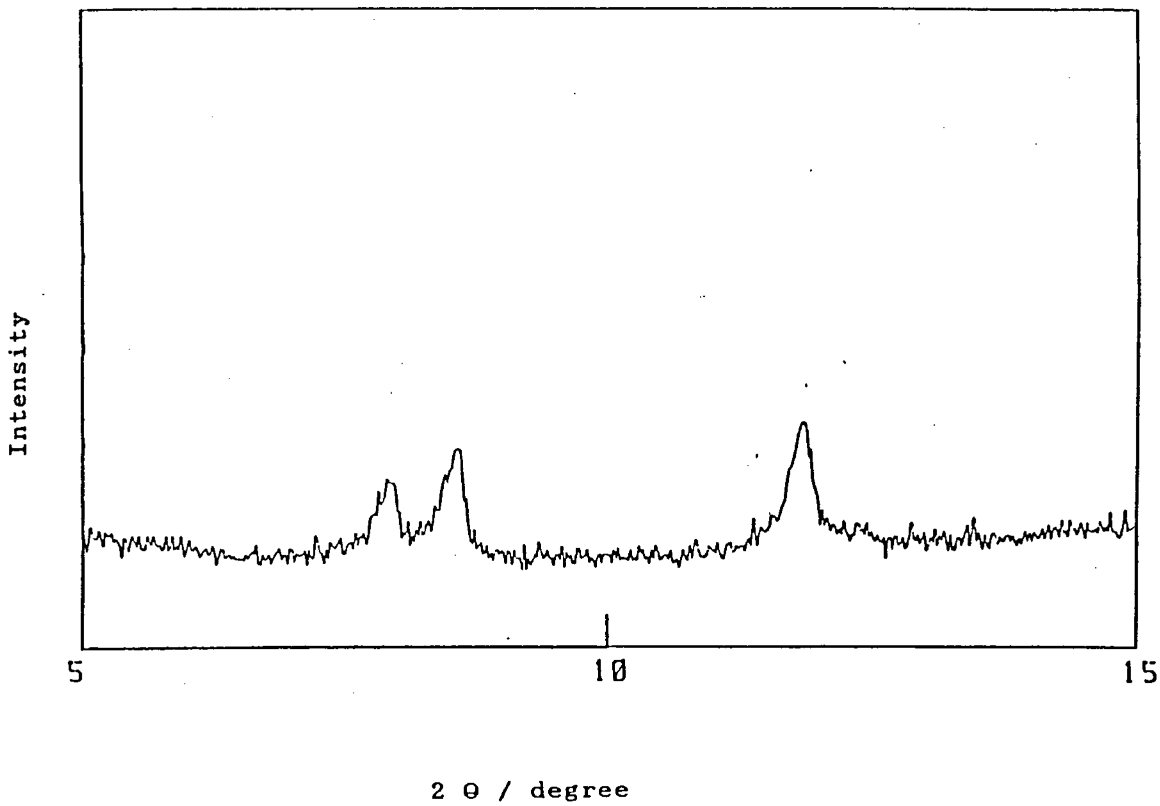


Fig.3

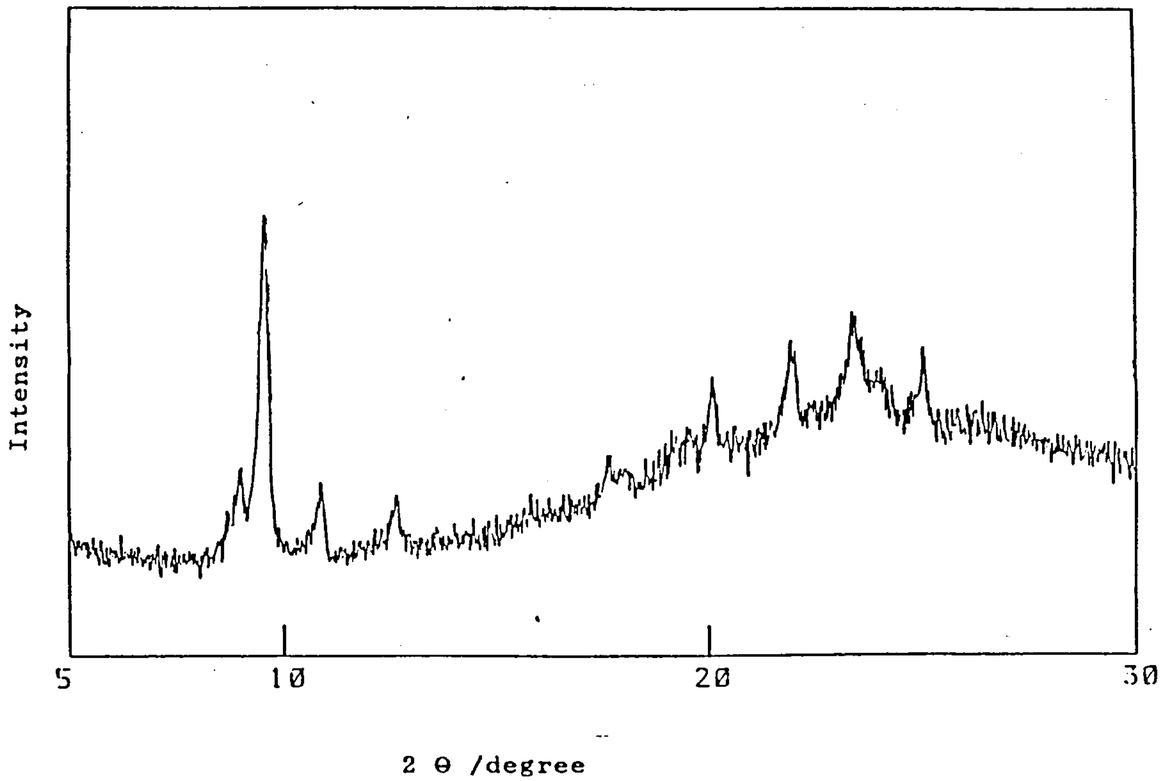


Fig.4

European Patent Office

# EUROPEAN SEARCH REPORT

Application Number

EP 91 81 0097

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-61 426 (CIBA-GEIGY)<br>* page 12, paragraph 3 - page 13, paragraph 3; claims 1-5 *<br>--- | 1-10 | C09K11/06<br>H05B33/14<br>C07D487/04<br>C09B57/00<br>C08K5/3415 |
| X | EP-A-232 222 (CIBA-GEIGY)<br>* page 8, paragraph 2 - page 8, paragraph 5; claims 1-14 *<br>--- | 1-3,5,8,10 | |
| X,D | WO-A-9 001 480 (RIEDEL DE HAEN)<br>* page 4 - page 5 *<br>--- | 1-10 | |
| X | DE-A-3 713 459 (LANGHALS)<br>* the whole document *<br>--- | 1-10 | |
| X | DATABASE DERWENT WORLD PATENT INDEX<br>AN 91-026391 "Electroluminescent element..."<br>&JP A 2296891( RICOH)<br>*abstract*<br>----- | 1-10 | |
| | | | TECHNICAL FIELDS SEARCHED (Int. Cl.5 ) |
| | | | C09K<br>H05B<br>C07D<br>C09B<br>C08K |

The present search report has been drawn up for all claims

| Place of search | Date of completion of the search | Examiner |
|---|---|---|
| THE HAGUE | 26 SEPTEMBER 1991 | DROUOT M.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document